Europäisches Patentamt

European Patent Office

Office européen des brevets

(19)

(11) Publication number : **0 362 999 B1**

(12) **EUROPEAN PATENT SPECIFICATION**

(45) Date of publication of patent specification :
21.04.93 Bulletin 93/16

(51) Int. Cl.[5] : **A61K 37/36, A23K 1/165**

(21) Application number : **89308537.3**

(22) Date of filing : **23.08.89**

(54) Improved carcass quality.

(30) Priority : **24.08.88 AU 24/88**
**08.02.89 AU 2630/89**

(43) Date of publication of application :
**11.04.90 Bulletin 90/15**

(45) Publication of the grant of the patent :
**21.04.93 Bulletin 93/16**

(84) Designated Contracting States :
**AT BE CH DE ES FR GB GR IT LI LU NL SE**

(56) References cited :
**EP-A- 0 192 360**
**EP-A- 0 217 572**
**EP-A- 0 280 407**
**EP-A- 0 283 458**
**US-A- 4 765 980**
**ANIMAL PRODUCTION (SCOTLAND), vol. 46, no. 3, 1988, page 487; J. NOVAKOFSKI et al.: "Pig carcass and pork sensory characteristics for animals given daily injections of natural or recombinant porcine from 57 to 103 kg"**

(56) References cited :
**JOURNAL OF ANIMAL SCIENCE, vol. 66, no. suppl. 1, 1988, pages 280-281; E. KANIS et al.: "Effect of recombinant porcine somatotropin (rPST) treatment on carcass characteristics and organ weights of growing pigs"**
**JOURNAL OF ANIMAL SCIENCE, vol. 66, no. 8, 1988, pages 1928-1941; C.M. EVOCK et al.: "Pituitary porcine growth hormone (pGH) and a recombinant pGH analog stimulate pig growth performance in a similar manner"**

(73) Proprietor : **FIDELITY FOOD TECHNOLOGIES PTY. LTD.**
**616 St. Kilda Road**
**Melbourne, Victoria (AU)**

(72) Inventor : **Walker, Ian James**
**468, Balwyn Road North Balwyn 3104**
**Victoria (AU)**
Inventor : **Campbell, Roger Gregory**
**174 Hume Street Corowa 2646**
**New South Wales (AU)**

(74) Representative : **Harding, Richard Patrick**
**Arthur R. Davies & Co. 27 Imperial Square**
**Cheltenham GL50 1RQ (GB)**

EP 0 362 999 B1

Jouve, 18, rue Saint-Denis, 75001 PARIS

## Description

This invention relates to animal husbandry, and in particular to improving the quality of livestock carcasses.

The quality of meat products is critically related to livestock husbandry and feeding practices, and is an important factor in consumer choice. The desirability to the consumer of a particular type of meat depends upon such parameters as price, tenderness, and appearance.

Nutritional factors, including the perceived "healthiness" of the meat, are an increasingly important influence in this area, and among these a high ratio of lean meat to fat is important.

A high ratio of lean meat to fat is also advantageous in the processing of the meat, since extensive trimming of fat and disposal of fat is avoided.

In addition, a high ratio of lean to fat is advantageous for economy of feed conversion, in that typically greater than four times the feed requirement is necessary to deposit 1 kg of fat compared to that necessary to deposit 1 kg of lean tissue.

In many countries the nature of the food raw material and the genotype of the animal result in a relatively inefficient conversion of feed meat, and in an undesirably high proportion of fat both on the surface and within the muscle.

Attempts to improve the ratio of lean to fat have been made using conventional breeding strategies. These have often been expensive, long-term, and not without undesirable side effects, such as deterioration in meat quality and increase in susceptibility of the target animal to stress.

For example in pig farming, daily administration of growth hormone is known in the prior art to promote increased weight gain and improved carcass quality thereby enhancing the food conversion ratio. Economic projections indicate that by treating pigs with "growth hormone" the quality of meat improves in conjunction with the efficiency and costs of production. Such techniques are illustrated in co-pending Australian patents AU-B-599922 and AU-D-6 252286. Unfortunately, the supply of natural porcine pituitary hormones falls well short of demand and given the limitation imposed by species specificity other natural homologues cannot be used cheaply as substitutes.

It is an object of the present invention to overcome, or at least alleviate, one or more of the difficulties related to the prior art.

According to the present invention there is provided a method for the treatment of an animal to improve carcass quality and/or food conversion efficiency, which method includes

    providing

        an animal selected from the group consisting of entire male, female and castrated male pigs;

        an exogenous synthetic porcine growth hormone, analogues, derivatives, or fragments thereof;

and

    administering to the animal at a preselected liveweight and at a preselected generally constant dose, depending on the sex of the animal, said exogenous synthetic growth hormone, every second, third or fourth day.

Preferably the dose is approximately 0.06 to 0.10 mg/kg liveweight/day when the animal is a female or castrated male pig, and the dose is approximately 0.10 to 0.15 mg/kg liveweight/day when the animal is an entire male pig.

Preferably the source of porcine synthetic growth hormone is growth hormone in a unit dosage form.

It is to be clearly understood that the use of biologically active fragments, analogues, derivatives or metabolites of synthetic porcine growth hormone, and the veterinarily acceptably salts thereof, are within the scope of the invention.

Further, recombinant porcine growth hormone is preferred. A recombinant porcine growth hormone selected from methionyl AA1-190 and AA4-190 has been found to be suitable for most domestic animals including pigs, sheep and cattle.

The preparation of recombinant growth hormone methionyl AA1-190 and AA4-190 is described in EP-A-0280407. Methionyl AA1-190 is preferred.

Although it is well known that growth hormone (GH) plays an important part in the control of normal growth, its precise role in the control of growth and of metabolism in domestic animals is not well understood. However, it is known that generally the administration of exogenous GH tends to increase the growth rate in all species studied. The role of GH in domestic animals has been briefly reviewed (C.G. Scanes,: Monasanto Symposium on Present and Future Trends in Animal Nutrition and Feed Manufacturing Technology, Arkansas, 1983).

Pigs currently provide an increasingly important source of meat in a large number of countries. According to usual practice, weaner pigs weighing 25 to 30 kg are transferred to a grower shed. Growth rate is relatively constant between 30 and 90 kg, but feed consumption increases significantly. Thus the economy of feed conversion progressively deteriorates as the animals approach the slaughter weight of approximately 90 to 110 kg. During the latter growth phase (60 to 110 kg), the carcass develops its final characteristics, and an increasing proportion of the weight gain is represented by fat. Since the energy requirement for production of fat is approximately 2.4 times greater than that for the production of lean tissue, it is clearly advantageous if a metabolic shift toward production of lean tissue can be achieved.

Several studies have investigated the effect of GH in pigs, with considerable variation in results. Turman and Andrews (J. Animal Sci. (1955) 44 7) injected 45 kg pigs daily with porcine GH, and found that treated pigs showed more efficient feed conversion and had leaner carcasses than controls, however, no increase in total weight gain was observed. Moreover, four out of eleven treated pigs died during the experiment as a result of liver and kidney degeneration. Another study (K.D. Lind, R.D. Howard, D.H. Kropf and B.A. Koch: J. Animal Sci. (1968) 27 1763) showed no effect on 72 kg pigs following treatment with porcine GH.

In a more detailed series of experiments, pigs of initial weight approximately 45 kg were treated daily with a range of GH concentration (L.J. Machlin: J. Animal Sci. (1972) 35 794), until they attained a weight of approximately 95 kg. Daily intramuscular injection of 0.22 or 1.10 mg porcine GH per kg bodyweight resulted in high mortality, with liver and kidney degeneration, gastric haemorrhage, oedema and arthritis. Doses of 0.033, 0.066 or 0.132 mg/kg/day resulted in improved average daily weight gain, efficiency of feed conversion, and lean/fat ratio, although the significance of the differences was variable. Furthermore, in 95 kg pigs fed a restricted high protein diet, GH treatment at 0.13 mg/kg/day improved the rate of weight gain compared to untreated controls.

More recently, it has been found that daily intramuscular injection for 30 days of 0.022 mg GH/kg liveweight in male pigs initially weighing 32 kg increased the growth rate by about 10%, and improved feed efficiency. There was no difference between treated and control groups with respect to dressing percentage, marbling score, loin eye area, or backfat thickness, while percentage lipid in the longissiums muscle was increased. There were no adverse effects of treatment on the animals.

We have now found that supplementing growth hormone levels in pigs results in improved carcass quality, as assessed by backfat thickness, fat and lean percentage, or loin eye area, optionally together, with improved feed conversion efficiency.

No deleterious effect upon the health of the animals was observed, and this was confirmed by pathological examination of all the body tissues of the animals.

When the animal to be treated is a pig, the administration of synthetic porcine growth hormone is preferably initiated at a liveweight of approximately 35 to 100 kg, more preferably 50 to 100 kg and most preferably about 60 kg.

Within the preferred initiation range the improvement in carcass quality is essentially similar to that achieved over the longer period. A greater relative improvement in feed conversion efficiency is noted.

Improvement in carcass quality can be measured as an overall increase in lean tissue content together with an increase in the proportion of lean tissue content may be attributed to an increased overall growth rate, an anabolic effect of a combination thereof.

The increase in the proportion of lean tissue to fat tissue may be attributed both to the anabolic effect and a decrease in fat tissue content.

Specifically, we have surprisingly found that there is a previously unsuspected critical interaction between dose of GH, the time of commencing treatment, the length of treatment, dietary energy supply, and sex of animal. The improved carcass quality in female and castrated male pigs was particularly unexpected, since normally they lay down fat more readily than entire males and produce an inferior carcass.

Our studies have shown that treatment according to the present invention enhances the growth rate in treated animals relative to controls. Moreover, the carcass quality is greatly improved and the feed consumption of the animals is lower, as a result of the more efficient utilisation of feed by animals treated with GH. At supra-optimal doses of GH, growth rate is not further enhanced, as apparently an appetite-suppressing effect can become limiting.

Results are essentially similar in terms of carcass quality if GH is given when the pigs initially weigh 50 to 60 kg to those found when treatment is given over a longer period, for example from 35 kg. Surprisingly, it is not necessary to increase the dose rate of GH in order to attain the same improvement in carcass quality over the shorter period. However, an improvement in feed efficiency is more apparent in treated animals over the 35 to 60 kg range.

We have found a striking increase in the proportion of lean meat in treated pigs. In a carcass of 60 to 65 kg dressed weight, for example, this represents an increase of 5 to 6 kg in the amount of meat, especially in the commercially valuable parts of the carcass. The economic desirability of such an improvement is clear.

As discussed above, it has surprisingly been found that within specified parameter ranges for particular animals an increase in lean tissue content together with an increase in the proportion of lean tissue to fat tissue may be achieved.

Previously the utilisation of synthetic porcine growth hormones has been extremely expensive and time consuming since it has required daily administration of unit dosage amounts of synthetic porcine growth hormone for a period of approximately 30 days or more.

However it has been surprisingly found that altering the periodicity of dosing provides a significant improvement in growth performance compared to controls. Whilst we do not wish to be restricted by theory, it is postulated that the modification of periodicity of dosing leads to alteration of the relative, anit-lipogenic and protein stimulatory effects of synthetic growth hormone on adipose and muscle tissue respectively. It is further postulated that high doses of synthetic growth hormone given daily depress lipogenesis and feed intake to such an extent that the associated improvement in growth rate is generally only small and often undetectable.

By altering periodicity to every second, third or fourth day administration, that is at intervals of 1, 2 or 3 days, it is postulated that the protein stimulatory effect, which is mediated via another hormone (insulin-like growth factor 1), is maintained but the anilipogenic effect which is direct is reduced.

Preferably the dose is approximately 10 mg of exogenous synthetic porcine growth hormone administered on every second, third or fourth day for a period of approximately 10 to 30 days, more preferably 30 days.

Administration of a dose of approximately 10 mg every second day is particularly preferred to maximise growth rate. Administration every fourth day provides a significant improvement in growth rate compared with controls but not the same magnitude as administration more frequently. However compared with controls food conversion efficiency is also improved by a dosage regimen of every fourth day.

The preselected generally constant dose used may be a reduced dose relative to that normally used in the prior art. For example, where a dose rate of approximately 10 mg per day may be standard throughout the industry, the dose may be reduced to approximately 50% or less of the standard dosage. It has surprisingly been found that such a reduction in dose amount has a similar and even enhanced effect on growth rate compared to controls at the high amounts described above. In addition feed conversion efficiency may be significantly improved.

Accordingly, the combination of alteration of dose and periodicity of dosage may be used to alter the relative effects of synthetic growth hormone on growth rate, feed conversion efficiency and carcass quality (fat levels). Thus it is possible to tailor the treatments to different types (genotype strains and sexes) of pigs.

In a development of the present invention there is provided a method of further increasing the growth performance of an animal to be treated, which method includes subsequently continuing administering the exogenous synthetic porcine growth hormone at an increased dose daily for a further preselected period.

For example, the exogenous synthetic porcine growth hormone may be administered to the animal initially at a dose of approximately 4 to 8 mg at intervals of 2 or 3 days for a period of approximately 10 to 25 days, and subsequently administration may be continued at an increased dose of approximately 6 to 10 mg daily for a further period of approximately 5 to 15 days.

It has surprisingly been found that the dosage regimen described above may lead to a further significant improvement in growth rate and food conversion efficiency over the total growth period whilst the later period of daily administration of synthetic growth hormone may lead to a further significant reduction in fat levels thus providing major improvements in carcass quality prior to slaughter.

Preferably the initial period of treatment extends for 15 to 20 days. The later period of full dose administration preferably extends for about 10 days.

The present invention also provides an animal carcass or part thereof whenever prepared according to the methods described above.

The methods of treatment described above may be provided utilising a veterinary composition including an aqueous buffer solution of approximately 3.3 to 6.6 mg/ml of a source of synthetic porcine growth hormone, analogues, derivatives or salts thereof.

Preferably the synthetic porcine growth hormone may be provided in a sustained-release form. Any suitable delivery system may be used which will provide sustained release. For example the hormone may be provided in a plurality of at least partially soluble microcapsules embedded in an at least partially soluble carrier.

The at least partially soluble carrier may be a polymeric article. The polymeric article may take the form of an implant or bolus.

A polymer which will function as an adjuvant for the synthetic growth hormone may be used. A water-soluble polymer may be used. The polymer article may preferably degrade in approximately 8 to 24 hours after entering the body of the animal. A polymer of the polyvinyl pyrrolidone type may be used. A polyvinyl pyrrolidone polymer or copolymer may be used. The polymer should be selected to provide sufficient impact strength to withstand the impact of the selected delivery system. Other standard compounding ingredients may be incorporated into the polymer matrix. Such compounding ingredients may include fillers and extenders. The polymer matrix may further include other active ingredients. Antibiotics, dietary supplements or drenches may also be included.

The polymeric article may be formed in any suitable manner. The polymeric article may be formed utilising an injection moulding technique.

The plurality of microcapsules may be incorporated into the polymeric article during the polymerization step thereof. Alternatively, the microcapsules may be incorporated at the molding stage. For example the polymeric article may be compressed into a desired shape utilising tableting technology. A tablet press may be used. The microcapsules and polymer may be mixed prior to moulding.

The microcapsules may be formed from any suitable at least partially soluble polymeric material. A polyester polymer may be used. Polymers and copolymers of -hydroxy acids and derivatives thereof are preferred.

The microcapsules may be formed from a first polymer or copolymer of glycolic acid, lactic acid, a derivative thereof or mixtures thereof having a relatively low molecular weight and a second polymer or copolymer of glycolic acid, lactic acid, a derivative thereof, or mixtures thereof having a relatively high molecular weight. More preferably the plurality of biodegradable microcapsules are formed in at least two particle sizes.

The plurality of biodegradable microcapsules include microcapsules having a relatively short degradation rate, a medium-term degradation rate or a relatively long degradation rate or a mixture thereof. As discussed above, degradation rates, and in turn the rate of release of the physiologically active ingredients incorporated therein, may be modified by utilising differing polymeric compositions and/or by modifying the molecular weight of the polymers used.

The synthetic porcine growth hormone may be encapsulated within the microcapsules in any suitable manner. The encapsulation process may include mixing the polyester or copolyester with the synthetic porcine growth hormone in a suitable solvent, and causing the polyester the precipitate.

The present invention will now be more fully described with reference to the accompanying examples. It should be understood however, that the description following is illustrative only and should not be taken in any way as a restriction on the generality of the invention described above.

The invention will be illustrated by reference to the following non-limiting examples.

The abbreviations used herein are defined as follows:

| | |
|---|---|
| GH | Growth hormone |
| ADG | Average daily liveweight gain (g/day) |
| FCR | Feed conversion ratio (kg feed/kg liveweight gain) |
| ADFC | Average daily feed consumption (kg/day) |
| P2 Backfat | Depth of backfat at the P2 position (mm) |
| Lean tissue % | Percentage of eviscerated carcass with head off remaining after fat and bones have been removed |
| Dressing (%) | Ratio of hot carcass weight to final liveweight prior to slaughter. |

In each experiment, porcine GH, was dissolved in buffer containing 0.05 M $Na_2CO_3$ and 0.05 M $NaHCO_3$, pH 10 to 11, and administered by intramuscular injection. Control animals received intramuscular injections of the same buffer containing 1% lactose. Unless otherwise stated, the pigs were fed _ad libitum_ on a standard high energy diet supplying 14.3 MJ/kg. Treatment commenced when the animals were approximately the weight stated in each case, and continued until they attained their slaughter weight. The animals were deprived of food and water for 24 hours before slaughter to enable an empty liveweight to be measured.

EXAMPLE 1

Comparison of effect of treatment with natural and recombinant growth hormone.

Table 1 illustrates that the results achieved with female pigs differ only marginally whether a natural or recombinant porcine growth hormone is used for treatment. Overall weight gain is greater with recombinant pGH than with natural pGH.

## TABLE 1

Effect of daily GH treatment on female pigs initially weighing 60 kg. The pigs were fed *ad libitum* and slaughtered when a weight of 90 kgs was reached.

|  | | pGH | |
| --- | --- | --- | --- |
|  | Control | Natural | Recombinant (AA1-190) |
| Number | 8 | 8 | 8 |
| Average daily gain ADG (gm) | 863 | 1049 | 1109 |
| Feed conversion effiency FCR | 3.50 | 2.54 | 2.57 |
| P2 backfat (mm) | 18.9 | 15.7 | 17.7 |
| Dissected lean: | | | |
| Weight per side (kg) | 17.8 | 18.1 | 18.8 |
| Lean Tissue % | 55.4 | 58.8 | 58.8 |

EXAMPLE 2

The abbreviations used herein are defined as follows:

pGH    porcine growth hormone (synthetic)

g/d      gram per day

Unless otherwise stated, the pigs were fed *ad libitum* on a standard high energy diet supplying 14.3 MJ/kg.

EP 0 362 999 B1

TABLE 2

Effects of pGH dose and frequency of administration between 60 and 90 kg on the growth performance and carcass P2 fat thickness of female pigs.

| pGH dose (mg/pig) | Frequency of administration | Growth rate (g/d) | Feed:gain | P2 (mm) |
|---|---|---|---|---|
| 0.0 | Control | 920 | 3.06 | 19.3 |
| 5.0 | Daily | 1080 | 2.41 | 14.6 |
| | 2nd day | 1040 | 2.54 | 15.7 |
| | 3rd day | 1025 | 2.66 | 17.3 |
| | 4th day | 1000 | 2.71 | 17.6 |
| 10.0 | Daily | 940 | 2.34 | 12.4 |
| | 2nd day | 1102 | 2.27 | 12.9 |
| | 3rd day | 1007 | 2.54 | 16.2 |
| | 4th day | 970 | 2.72 | 16.9 |

The experimental results are summarised in Table 2. The experiment involved two porcine Growth Hormone (pGH) doses (5 and 10mg) administered daily, every second day, every third day or every fourth day to female pigs growing from 60 to 90 kg. Ten control pigs (injected with a buffer solution every second day) and six pigs on each of the dose x administration treatments (2 x 4 factorial arrangement) were used.

They show that by administering the low dose (5 mg) every second or third day we can achieve the same growth rate improvement over controls as daily administration (current technology for pigs) but reduce the amount of material required by 1/2 and 2/3 respectively. The 5 mg dose every second day also enables a similar improvement in feed:gain to be achieved as daily administration.

At the higher dose (10 mg) changing administration from daily to every second day actually results in more rapid gain and improvement in daily gain over controls than daily administration and the every second day administration treatments give slightly higher proportional improvements in feed:gain compared to daily administration of either 10 mg or 5 mg porcine growth hormone.

Based on these results the preferred employment of the present invention would be to use 5 mg pGH every second day or alternatively a higher dose every third day to obtain maximum improvement in terms of growth rate of feed:gain and considerably lower cost than presently required with daily administration. On a less restrictive basis the preferred technique would be to alter dose and frequency of dosing to maximise improvements in growth rate, feed:gain and carcass quality with respect to the type of pig involved (would include starting weight, strain, sex and genotype).

Results further show that 10 mg pGH administered every second day results in an improvement in growth rate compared with daily administration due to a smaller antilipogenic effect during the earlier stages of growth, and an improvement in growth performance and surprisingly a better improvement in carcass P2 fat thickness than 5 mg administered daily, and similar P2 fat thickness as 10 mg administered daily. This finding has obvious implications with respect to economy of use of PST and ease of administration (every second rather than daily administration of 5 mg/d equivalent).

The findings also show that the effect less frequent dosing treatments in reducing feed: gain and P2 fat

7

thickness declined relative to daily administration. This was expected due to the fact that the antilipogenic effects of the less frequent dosing treatments would be expected to decline in the later stages of growth because the dosage per unit body weight is continually declining at a time when fat deposition is becoming an increasingly greater proportion of total growth resulting in an increased fat: protein ratio and as such less efficient growth (higher feed:gain) and fatter carcasses.

Nevertheless, this did not occur with the 10 mg dose administered every second day probably because at this dose fat deposition was adequately depressed or alternatively protein deposition enhanced to such an extent that the fat:protein ratio was similar throughout the whole growth period (but probably different during earlier and later growth, prior to and subsequent to 14 days) as that of pigs administered 10 mg pGH daily.

The results also show that although the second, third and fourth day treatments at 5mg/pig and third, and fourth day treatments at 10mg/pig resulted in proportionately smaller improvements in feed:gain and carcass fatness they were all significantly better than the controls in both respects.

It is here that the findings have other implications - because under certain circumstances it may be more economical to use less pGH and not necessarily achieve maximum improvements in growth and carcass quality. For example if you were to treat very lean genotypes or alternatively boars with pGH you may not wish to reduce carcass P2 fat thickness by 30-40% to achieve a target fat level which may only require a 20% reduction in these leaner animals compared with fatter genotypes and female pigs. For instance, administering 10 mg of pGH/pig every third day for 30 days would, on the basis of these results, improve growth rate 11.4% and reduce feed:gain (feed usage) and carcass P2 fat thickness 13.1 and 10.4% respectively and require a total of only 50 mg of pGH. The data show therefore that dose and dose frequency can be used as tools to tailor pGH technology for different breeds, genotypes and different economical circumstances (depending on price of pGH and relative returns for improvements in growth rate, feed:gain and carcass fatness).

EXAMPLE 3

Because the effects of pGH administration on growth performance and carcass fat thickness declined as did frequency of dosing, the second experiment was conducted to investigate an alternative dosing strategy involving three doses (4, 6 and 8 mg/pig) and various dosing frequencies for a 30 day period commencing at 60 kg. The basic plan was to investigate if the partial loss of the antilipogenic effect during the later stages of growth with the less frequent dosing practices could be combated by imposing a daily administration treatment during the last 10 days of the 30 day period when fat deposition would normally be increasing in untreated pigs. The various treatments imposed to test this possibility are set out below:

Treatments:

(1) Control - Daily buffer injection for 30 days
(2) 4.0 mg pGH/pig - Daily for 30 days
(3) 4.0 mg pGH/pig - second day for 30 days
(4) 4.0 mg pGH/pig - second day for 20 days and 6 mg daily for 10 days
(5) 6.0 mg pGH/pig - Daily for 30 days
(6) 6.0 mg pGH/pig - second day for 30 days
(7) 6.0 mg pGH/pig - third day for 30 days
(8) 6.0 mg pGH/pig - third day for 20 days and 6mg daily for 10 days
(9) 8.0 mg pGH/pig - Daily for 30 days
(10) 8.0 mg pGH/pig - third day for 20 days
(11) 8.0 mg pGH/pig - third day for 20 days and 6 mg daily for 10 days
The total amount of pGH used per pig for the 30 day period was:

| Treatment | pGH (mg/pig) |
|---|---|
| 1 | 0.0 |
| 2 | 120 |
| 3 | 60 |
| 4 | 80 |
| 5 | 180 |
| 6 | 90 |
| 7 | 60 |
| 8 | 102 |
| 9 | 140 |
| 10 | 80 |
| 11 | 116 |

Seven female pigs were allocated to each treatment at 60 kg liveweight. They were offered a single diet adlibitum and liveweight gain and feed intake was recorded weekly. Each pig was killed after 30 days treatment and carcass fat thickness measured at the P2 position (6.5 cm from the mid-line at the level of the last rib).

The results for growth performance and carcass P2 fat thickness are presented in Table 3.

Because of an outbreak of comphilobacter during the experiment a number of pigs exhibited severe diarrhoea and had to be withdrawn from the experiment and treated with antibiotics. The performance of these animals was not included in the results. The number of animals completing the experiment is shown in brackets in Table 3.

## TABLE 3

Effect of pGH dose and dosing frequency for 30 days on the growth performance and carcass P2 fat thickness of female pigs commencing at 60 kg liveweight.

| pGH dose (mg/pig) | Dosing 0-20d | Frequency 20-30d | | Growth rate (g/d) | Feed:gain | P2 (mm) |
|---|---|---|---|---|---|---|
| 0.0 | Control | | (7) | 944 | 2.91 | 21.5 |
| 4.0 | daily | daily | (6) | 966 | 2.42 | 15.5 |
| | 2nd d | 2nd d | (5) | 986 | 2.61 | 16.9 |
| | 2nd d | 6mg/d | (6) | 998 | 2.32 | 13.9 |
| 6.0 | daily | daily | (6) | 990 | 2.30 | 14.4 |
| | 2nd d | 2nd d | (6) | 1123 | 2.36 | 16.0 |
| | 3rd d | 3rd d | (5) | 1050 | 2.52 | 16.8 |
| | 3rd d | 6mg/d | (6) | 1050 | 2.27 | 15.0 |
| 8.0 | daily | daily | (5) | 990 | 2.30 | 13.7 |
| | 3rd d | 3rd d | (5) | 1001 | 2.60 | 17.4 |
| | 3rd d | 6mg/d | (6) | 1045 | 2.24 | 14.5 |

The overall findings from the study outlined in Example 3 are:

(i) That regardless of dose or dosing frequency, pGH administration improved growth performance and reduced P2 carcass fatness compared to controls.

(ii) Confirmation of previous claim that at higher doses pGH administration every second day improves growth rate proportionately more than daily administration but gives similar improvement in feed:gain. This is evident from the 6mg pGH treatments although there was some loss of the antilipogenic effect comparing the P2 values for pigs dosed every second day with those dosed daily.

(iii) That at the three doses tested the implementation of less frequent dosing (second or third day) for 20 days followed by daily dosing (6 mg/pig) during the last 10 days of the 30 day period resulted in carcass fat thickness and feed:gain for the whole period returning to the level of pigs administered pGH daily for the 30 day period.

Surprisingly, at the lowest dose tested (4 mg/pig) dosing pigs every second day for 20 days followed by daily dosing with 6 mg/pig for the last 10 days resulted in slight improvements in feed:gain and carcass P2 fat thickness compared with daily administration for the 30 day period.

Overall, results show that a preferred dosage regimen comprising infrequent dosing for 20 days (second or third day) followed by daily dosing for last 10 days enables similar improvements in growth performance and carcass quality (less fat) to be achieved as daily dosing but significantly reduces pGH usage.

Results are presumably achieved because higher and more frequent dosing during later stages of growth enables the marked increase in fat deposition which normally occurs during this period to be depressed resulting in leaner and more efficient growth.

**Claims**

1. A method for the treatment of an animal to improve carcass quality and/or food conversion efficiency, which method includes
   providing
   an animal selected from the group consisting of entire male, female an castrated male pigs;
   an exogenous synthetic porcine growth hormone, analogues, derivatives, or fragments thereof; and
   administering to the animal at a preselected liveweight and at a preselected generally constant dose, depending on the sex of the animal, said exogenous synthetic growth hormone, every second, third or fourth day.

2. A method according to claim 2, wherein the exogenous synthetic porcine growth hormone is a recombinant porcine growth hormone selected from methionyl AA1-190 and AA4-190.

3. A method according to claim 3, wherein the administration is initiated at a liveweight of approximately 50 to 100 kg.

4. A method according to claim 4, wherein the dose is approximately 10 mg of exogenous synthetic growth hormone administered on every second, third or fourth day for a period of approximately 10 to 30 days.

**Patentansprüche**

1. Verfahren zur Behandlung eines Tieres zur Verbesserung der Qualität des Schlachtkörpers und/oder des Futterverwertungsgrades, welches umfaßt:
   Bereitstellung eines Tieres, ausgewählt aus der Gruppe der nicht kastrierten männlichen, weiblichen und kastrierten männlichen Schweine;
   eines exogenen synthetischen Schweinewachstumshormons, dessen Analogen, Derivaten oder Fragmenten; und
   Verabreichung des exogenen synthetischen Wachstumshormons an das Tier mit einem vorgewählten Lebendgewicht und mit einer vorgewählten, im allgemeinen konstanten Dosis, in Abhängigkeit von dem Geschlecht des Tieres, an jedem zweiten, dritten oder vierten Tag.

2. Verfahren nach Anspruch 1, worin das exogene synthetische Schweinewachstumshormon ein rekombinantes Schweinewachstumshormon darstellt, das aus Methionyl-AA1-190 und AA4-190 ausgewählt ist.

3. Verfahren nach Anspruch 2, worin mit der Verabreichung bei einem Lebengewicht von etwa 50 bis 100 kg begonnen wird.

4. Verfahren nach Anspruch 3, worin die Dosis etwa 10 mg exogenes synthetisches Wachstumshormon beträgt, die an jedem zweiten, dritten oder vierten Tag über einen Zeitraum von etwa 10 bis 30 Tagen verabreicht wird.

**Revendications**

1. Procédé de traitement d'un animal en vue d'améliorer la qualité de la carcasse et/ou l'efficacité de la conversion de nourriture, procédé selon lequel :
   on prend :
   un animal choisi dans le groupe consistant en porcs entiers mâles, femelles, et mâles castrés ;
   une hormone de croissance porcine synthétique exogène, ses analogues, dérivés ou fragments ; et
   on administre tous les deux, trois ou quatre jours ladite hormone de croissance synthétique exogène à l'animal à un poids vif présélectionné et selon un dosage présélectionné globalement constant.

2. Procédé selon la revendication 1, dans lequel l'hormone de croissance porcine synthétique exogène est une hormone de croissance porcine recombinante choisie parmi le méthionyl AA1-190 et AA4-190.

3. Procédé selon la revendication 2, dans lequel on commence l'administration à un poids vif d'environ 50 à 100 kg.

4. Procédé selon la revendication 4, dans lequel le dosage est d'environ 10 mg d'hormone de croissance synthétique exogène administré tous les deux, trois ou quatre jours pendant une période d'environ 10 à 30 jours.